# EUROPEAN PATENT SPECIFICATION

(11) **EP 2 722 007 B1**
(45) Date of publication and mention of the grant of the patent: **26.07.2017**
(21) Application number: 12800541.0
(22) Date of filing: 13.06.2012
(51) Int. Cl.: A61B 10/00, G01N 1/20

(54) **URINE SAMPLER**
URINPROBENNEHMER
ÉCHANTILLONNEUR D'URINE

(30) Priority: 16.06.2011 CN 201120203292 U
(43) Date of publication of application: 23.04.2014
(73) Proprietor: Cao, Shujun, Wuxi, Jiangsu 214028 (CN)
(72) Inventor: Cao, Shujun, Wuxi, Jiangsu 214028 (CN)
(74) Representative: Hanna Moore + Curley
(86) International application number: PCT/CN2012/076835
(87) International publication number: WO 2012/171458

(56) References cited:
- CN-U- 201 508 292
- CN-U- 201 508 292
- CN-U- 202 136 355
- CN-Y- 201 242 513
- US-A- 3 680 543
- US-A- 4 494 581
- US-A- 5 902 294
- US-A1- 2002 132 369
- US-A1- 2005 010 189
- US-B1- 6 409 971

## Description

### FIELD OF THE INVENTION

The invention relates to a medical test auxiliary tool, in particular, a urine sampler.

### BACKGROUND OF THE INVENTION

Currently, most of the medical urine samplers for use in hospitals are disposable cup or disposable cup together with a test tube. It is not convenient for urine collection by the patient and easy to contaminate the patient. Besides, the urine cup without a cover is likely to have urine spills, more likely when pouring urine into the test tube. It is neither convenient nor sanitary for use. The spill issue will lead to inaccurate urine sampling in quantitative sense. A urine sampler according to the preamble of appended claim 1 is disclosed in US 2002/0132369. Other urine samplers are known, for example, from CN 201508292 U; US 6,409,971; US 4,494,581; US 2005/0010189; CN 201242513Y.

### SUMMARY OF THE INVENTION

The present invention has been made in view of the above-mentioned problems. Therefore, after research and improvement, the applicant provides a convenient and useful urine sampler, which is both sanitary and easy for use and helps with accurate sampling due to unlikely spills.

In order to solve the above-mentioned technical issues, the present invention provides a urine sampler as defined in claim 1, which will now be described as follows:
A urine sampler, comprising a urine cup, a switch tube base and a test tube, wherein the said urine cup is funnel-shaped with a connecting tube at the lower end; the said connecting tube is sleeved into the said switch tube base and the mouth of the said test tube is in threaded connection with the said switch tube base.

Further, the said switch tube base includes a right-angled tube base which is composed of a tube base and tube sleeve in right-angle connection, and a switch tube which includes a switch section with a through-hole on its side wall and a joint section with internal threads, the switch section of the said switch tube is sleeved into the tube sleeve of the said right-angled tube base rotationally. In a preferred embodiment, the said connecting tube of the urine cup is tapered and the said connecting tube is sleeved into the tapered tube base of the said right-angled tube base.

Further, the side wall of the said urine cup may provide scales.

Further, the said urine cup may provide guiding mouth that extends outward.

### Technical benefits of the invention:

The urine sampler disclosed in the present invention, is simple and compact in structure, easy for operation and low in manufacturing cost. When in use, the test tube works as handle and the urine in the urine cup can be directly guided into the test tube through the switch tube base so that the sample is unlikely to spill out, not only achieving accurate sampling but also avoiding the patient's exposure to urine contamination. The invention is thus hygienic and easy to use.

### BRIEF DESCRIPTION OF THE DRAWINGS

FIG. 1 is a front view of the present invention.
FIG.2 is a three-dimensional structure schematic drawing of the present invention.

### DETAILED DESCRIPTION OF PARTICULAR EMBODIMENT

With reference to the accompanying drawings, the embodiment will now be described to illustrate the invention.

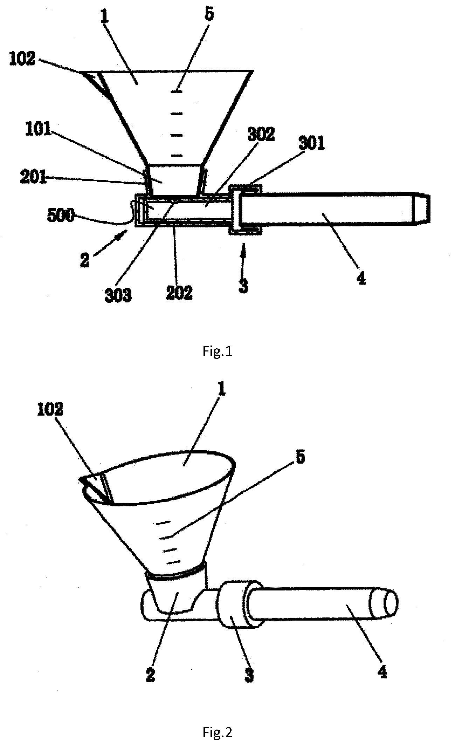

As is shown in FIG. 1 and FIG. 2, the present invention comprising urine cup 1, switch tube base 500 and test tube 4. Urine cup 1 is funnel-shaped with connecting tube 101 at its lower end. The side wall of urine cup 1 provides scale 5. Urine cup 1 provides guiding mouth 102 that extends outward. The switch tube base 500 includes right-angled tube base 2 which is composed of tube base 201 in right-angle connection with tube sleeve 202; and switch tube 3 comprising switch section 302 with a through-hole on its side wall and joint section 301 with internal threads, wherein joint section 302 of switch tube 3 is sleeved into the tube sleeve 202 of the said right-angled tube base 2 rotationally. Connecting tube 101 of urine cup 1 is sleeved into the tube base 201 of right-angled base 2 of the switch tube base 500. The tube mouth of test tube 4 is connected to the joint section 301 of switch tube 3 of switch tube base 500 by threaded connection. In this embodiment example, the connecting tube 101 of urine cup 1 is tapered and the said connecting tube 101 is sleeved into the tapered tube base 201 of the said right-angled tube base 2.

Switch tube base 500 functions as a switch, enabling control of the urine flow into test tube 4. When rotating switch tube 3 so as to align through-hole 303 of switch section 302 with the connecting tube 101 of urine cup 1, the urine in urine cup 1 can flow into test tube 4 through switch tube 3. When in need of urine sampling, rotate switch tube 3, and turn through-hole 303 of switch section 302 downward so that there is no connection between urine cup 1 and test tube 4 for the convenience of urine sampling; when the urine in urine cup 1 reaches the specified scale 5, finish the sampling, rotate switch tube 3 and align through-hole 303 of switch section 302 with connecting tube 101 of urine cup 1 so that the urine flows into test tube 4. After the urine enters test tube 4, rotate the switch tube 3 again to close test tube 4 so as to avoid urine spills. During the sampling process, test tube 4 works as the handle for the patient to hold the urine cup and avoid patient's exposure to urine contamination.

## Claims

1. A urine sampler comprising a urine cup (1), a switch tube base (500) and a test tube (4), wherein the said urine cup is funnel-shaped with a connecting tube (101) at the lower end;
the said connecting tube is sleeved into the said switch tube base and the mouth of the said test tube is in threaded connection with the said switch tube base;
**characterized in that**
the said switch tube base includes a right-angled tube base (2) which is composed of a tube base (201) and a tube sleeve (202) in right-angle connection, and a switch tube (3) which includes a switch section (302) with a through-hole (303) on its side wall and a joint section (301) with internal threads, the switch section of the said switch tube is sleeved into the tube sleeve of the said right-angled tube base rotationally.

2. The urine sampler according to claim 1, wherein the said connecting tube of the urine cup is tapered and the said connecting tube is sleeved into the tapered tube base of the said right-angled tube base.

3. The urine sampler according to claim 1, wherein the side wall of the said urine cup provides scales (5).

4. The urine sampler according to claim 1, wherein the said urine cup provides a guiding mouth (102) that extends outward.

## Patentansprüche

1. Urinprobennehmer, umfassend einen Urinbecher (1), eine Wechselrohrbasis (500) und ein Testrohr (4),
wobei der Urinbecher trichterförmig mit einem Verbindungsrohr (101) am unteren Ende ist;
das Verbindungsrohr in die Wechselrohrbasis gesteckt ist und die Mündung des Testrohrs in Gewindeverbindung mit der Wechselrohrbasis steht;
**dadurch gekennzeichnet, dass**
die Wechselrohrbasis eine rechtwinklige Rohrbasis (2), die aus einer Rohrbasis (201) und einer Rohrhülse (202) in rechtwinkliger Verbindung gebildet ist, und ein Wechselrohr (3) aufweist, das einen Wechselabschnitt (302) mit einer Durchgangsöffnung (303) an seiner Seitenwand und einen Verbindungsabschnitt (301) mit Innengewinde aufweist, wobei der Wechselabschnitt des Wechselrohrs drehend in die Rohrhülse der rechtwinkligen Rohrbasis gesteckt ist.

2. Urinprobennehmer nach Anspruch 1, wobei das Verbindungsrohr des Urinbechers sich verjüngt und das Verbindungsrohr in die sich verjüngende Rohrbasis der rechtwinkligen Rohrbasis gesteckt ist.

3. Urinprobennehmer nach Anspruch 1, wobei die Seitenwand des Urinbechers Skalen (5) bereitstellt.

4. Urinprobennehmer nach Anspruch 1, wobei der Urinbecher eine Führungsmündung (102) bereitstellt, die sich nach außen erstreckt.

## Revendications

1. Échantillonneur d'urine comprenant un gobelet pour urine (1) une base de tube de commutation (500) et un tube à essai (4),
dans lequel ledit gobelet pour urine est en forme d'entonnoir avec un tube de liaison (101) au niveau de l'extrémité inférieure ;
ledit tube de liaison est manchonné dans ladite base de tube de commutation et l'embouchure dudit tube à essai est vissée avec ladite base de tube de commutation ;
**caractérisé en ce que**
ladite base de tube de commutation comprend une base de tube à angle droit (2) qui se compose d'une base de tube (201) et d'un manchon de tube (202) reliés par un angle droit, et un tube de commutation (3) qui comprend une section de commutation (302) avec un trou traversant (303) sur sa paroi latérale et une section de jointure (301) avec des filetages internes, la section de commutation dudit tube de commutation est manchonnée rotative dans le manchon de tube de ladite base de tube à angle droit.

2. Échantillonneur d'urine selon la revendication 1, dans lequel ledit tube de liaison du gobelet pour urine est conique et ledit tube de liaison est manchonné dans la base de tube conique de ladite base de tube à angle droit.

3. Échantillonneur d'urine selon la revendication 1, dans lequel la paroi latérale dudit gobelet pour urine fournit des graduations (5).

4. Échantillonneur d'urine selon la revendication 1, dans lequel ledit gobelet pour urine fournit une embouchure de guidage (102) qui se déploie vers l'extérieur.
